(19) Europäisches Patentamt
European Patent Office
Office européen des brevets

(11) **EP 3 188 774 B1**

(12) **EUROPÄISCHE PATENTSCHRIFT**

(45) Veröffentlichungstag und Bekanntmachung des Hinweises auf die Patenterteilung:
**27.11.2019 Patentblatt 2019/48**

(21) Anmeldenummer: **15763833.9**

(22) Anmeldetag: **03.09.2015**

(51) Int Cl.:
*A61M 5/145* (2006.01)        *A61M 1/16* (2006.01)
*A61M 5/36* (2006.01)        *G01N 7/00* (2006.01)

(86) Internationale Anmeldenummer:
**PCT/EP2015/001782**

(87) Internationale Veröffentlichungsnummer:
**WO 2016/034286 (10.03.2016 Gazette 2016/10)**

(54) **VERFAHREN ZUR BESTIMMUNG EINES SYSTEMKOMPRESSIBILITÄTSWERTES EINES MEDIZINISCHEN MEMBRANPUMPENANTRIEBS**

METHOD TO DETERMINE THE SYSTEMIC COMPLIANCE OF A MEDICAL MEMBRANE PUMP DRIVE

MÉTHODE POUR DÉTERMINER LA COMPLIANCE SYSTÉMIQUE DE L'ACTIONNEMENT D'UNE POMPE MEDICALE À MEMBRANE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.09.2014 DE 102014013152**

(43) Veröffentlichungstag der Anmeldung:
**12.07.2017 Patentblatt 2017/28**

(73) Patentinhaber: **Fresenius Medical Care Deutschland GmbH**
**61352 Bad Homburg (DE)**

(72) Erfinder:
• **HEDMANN, Frank**
**97332 Volkach (DE)**

• **HOCHREIN, Torsten**
**97478 Knetzgau (DE)**

(74) Vertreter: **Behr, Wolfgang**
**Lorenz Seidler Gossel**
**Rechtsanwälte Patentanwälte**
**Partnerschaft mbB**
**Widenmayerstraße 23**
**80538 München (DE)**

(56) Entgegenhaltungen:
**WO-A2-2013/176770        DE-A1- 19 919 572**
**DE-B3-102011 105 824**

EP 3 188 774 B1

**Beschreibung**

Die vorliegende Erfindung betrifft ein Verfahren zur Bestimmung eines Systemkompressibilitätswertes eines medizinischen Membranpumpenantriebs, sowie ein Verfahren zur Bestimmung eines Luftanteils und / oder einer Luftmenge in einer von einer Membranpumpe geförderten medizinischen Flüssigkeit.

Membranpumpen werden im Bereich der Medizintechnik, und insbesondere im Bereich der Dialysetechnik, oftmals zum Pumpen medizinischer Flüssigkeiten wie beispielsweise Dialysat oder Blut eingesetzt. Eine Membranpumpe weist dabei üblicherweise eine durch eine Membran abgeschlossene Pumpkammer auf, wobei durch Hineindrücken der Membran in die Pumpkammer Fluid aus der Pumpkammer herausgedrückt, und durch Herausziehen der Membran aus der Pumpkammer Fluid in die Pumpkammer eingesogen werden kann. Im Zusammenspiel mit entsprechenden Ventilen kann hierdurch Flüssigkeit durch die Pumpkammer gepumpt werden.

Die Pumpkammer ist dabei meistens in einem Disposable, beispielsweise einer Pumpkassette, angeordnet, welche an einen Membranpumpenantrieb angekoppelt wird. Der Membranpumpenantrieb weist dabei üblicherweise eine Antriebskammer auf, welche ebenfalls durch eine Membran abgeschlossen ist. Pumpkammer und Antriebskammer werden dann so aneinander gekoppelt, dass die Membran der Pumpkammer der Bewegung der Membran der Antriebskammer folgt.

Aus der WO 2013/176770 A2 ist eine Membranpumpe bekannt, welche pneumatisch angetrieben wird, indem die Antriebskammer abwechselnd mit Unterdruck, atmosphärischem Druck und Überdruck beaufschlagt wird. Dabei wird ein Verfahren zur Bestimmung der Kompressibilität der von der Membranpumpe geförderten medizinischen Flüssigkeit beschrieben, bei welchem die Antriebskammer bei geschlossener Pumpkammer nacheinander mit Überdruck und atmosphärischem Druck bzw. atmosphärischem Druck und Unterdruck beaufschlagt und die hierdurch hervorgerufene Volumenänderung der Pumpkammer bestimmt, was durch Einleiten von Schallwellen in die Pumpkammer erfolgt.

Bei einer Kolbenmembranpumpe steht die Antriebskammer dabei mit einer Kolben-Zylindereinheit hydraulisch in Verbindung. Durch Bewegen des Kolbens kann Hydraulikfluid in die Antriebskammer hineingedrückt bzw. aus dieser herausgesogen werden, was eine entsprechende Bewegung der Membran der Antriebskammer zur Folge hat. Eine solche Anordnung hat den Vorteil, dass der Pumpdruck durch eine entsprechende Ansteuerung bzw. Regelung des Drucks im Hydraulikteil kontrolliert werden kann. Weiterhin ermöglichen Membranpumpen eine einfache Bilanzierung der gepumpten Flüssigkeiten, da die Volumenänderung der Pumpkammer und damit die Flüssigkeitsverdrängung bei einem Pumphub der Volumenänderung der Steuerkammer (mit umgekehrten Vorzeichen) entspricht, wobei diese über die Position des Kolbens der Kolben-Zylinder-Einheit genau bestimmt werden kann.

Hierbei können jedoch Fehlerquellen auftreten: Zum einen können Luftansammlungen in der Pumpkammer dazu führen, dass die durch die Pumpkammer gepumpte Flüssigkeitsmenge nicht genau der Volumenänderung der Antriebskammer entspricht. Weiterhin kann aufgrund einer gewissen Systemkompressibilität des Membranpumpenantriebs die Volumenänderung der Steuerkammer von der durch die Bewegung des Kolbens der Kolben-Zylinder-Einheit bedingten Volumenänderung abweichen. Insbesondere kann dabei Luft, welche sich in der Hydraulikflüssigkeit ansammelt, zu einer gewissen Kompressibilität des Hydrauliksystems führen. Weiterhin können beispielsweise Schläuche, welche die Kolben-Zylinder-Einheit mit der Antriebskammer verbinden, eine gewisse Flexibilität aufweisen und sich daher bei erhöhtem Druck ausdehnen. Auch bei anderen Antriebsmechanismen kann es zu einer gewissen Systemkompressibilität kommen, welche die für die Bilanzierung erfassten Werte beeinflusst.

Aus der DE 19919572 A1 ist dabei ein Verfahren bekannt, durch welches der Luftanteil in der durch eine Pumpkammer gepumpten Flüssigkeit bestimmt werden kann. Hierzu wird die Pumpkammer zunächst gravimetrisch befüllt und der sich hierdurch ergebende Ausgangsdruck gemessen. Daraufhin werden die Absperrventile der Pumpkammer geschlossen, sodass sich ein in dieser eingeschlossenes Flüssigkeitsvolumen ergibt. Bei geschlossenen Absperrventilen wird dann die Kolben-Zylinder-Einheit betätigt, um das abgeschlossene Flüssigkeitsvolumen mit einem vorgegebenen Enddruck zu beaufschlagen. Die mit dieser Druckänderung einhergehende Volumenänderung des Flüssigkeitsvolumens in der Pumpkammer hängt dabei unmittelbar vom Anteil der Luft im eingeschlossenen Flüssigkeitsvolumen zusammen. Daher kann der Luftanteil anhand der durch die Druckdifferenz erzeugten Volumenänderung, welche über die Kolbenbewegung bestimmt wird, ermittelt werden. In der DE 19919572 A1 wird dabei der Einfluss der Systemkompressibilität des Membranpumpenantriebs durch eine fest vorgegebene Konstante berücksichtigt. Allerdings kann sich die Systemkompressibilität beispielsweise durch sich ansammelnde Luft im Hydraulikfluid während des Betriebs der Pumpe verändern, was in der DE 19919572 A1 unberücksichtigt bleibt.

Aus der DE 102011105824 B3 ist daher ein Verfahren bekannt, wie die Systemkompressibilität eines Membranpumpenantriebs bestimmt werden kann. Dabei wird die Systemkompressibilität der mit Gas gefüllten Pumpvorrichtung dadurch bestimmt, dass ein Start- und Enddruck mit einem Drucksensor eingeregelt und die zugehörigen Pumppositionen bzw. Drucksensorwerte aufgenommen werden. Aufgrund der Wertepaare wird die Federkonstante bestimmt, welche mit der Systemkompressibilität gleichgesetzt wird.

Gemäß einem ersten Aspekt ist es Aufgabe der vorliegenden Erfindung, ein verbessertes Verfahren zur Bestimmung eines Systemkompressibilitätswertes eines medizinischen Membranpumpenantriebs zur Verfügung zu stellen. Gemäß eines zweiten Aspektes ist es Aufgabe der vorliegenden Erfindung, ein verbessertes Verfahren zur Bestimmung eines Luftanteils und / oder einer Luftmenge in einer von einer Membranpumpe geförderten medizinischen Flüssigkeit zur Verfügung zu stellen. Weiterhin ist es Aufgabe der vorliegenden Erfindung, entsprechende Membranpumpenantriebe bzw. Blutbehandlungsmaschinen mit entsprechenden Membranpumpenantrieben zur Verfügung zu stellen, welche die erfindungsgemäßen Verfahren durchführen.

Gemäß dem ersten Aspekt umfasst die vorliegende Erfindung ein Verfahren zur Bestimmung eines Systemkompressibilitätswertes eines medizinischen Membranpumpenantriebs, bei welchem ein erstes und ein zweites Druckniveau angefahren und ein erster und zweiter Betriebsparameterwert des Membranpumpenantriebs erfasst werden, wobei der Systemkompressibilitätswert auf Grundlage der erfassten Betriebsparameterwerte bestimmt wird. Gemäß der vorliegenden Erfindung stützt sich die Membran des Membranpumpenantriebs dabei während der Bestimmung des Systemkompressibilitätswertes an einer starren Fläche ab. Insbesondere kann sich die Membran des Membranpumpenantriebs dabei an einer starren Fläche abstützen, während der dem ersten und zweiten Druckniveau entsprechende erste und zweite Betriebsparameterwerte erfasst wird.

Die vorliegende Erfindung berücksichtigt dabei, dass ein Membranpumpenantrieb eigentlich kein geschlossenes, in seinem Gesamtvolumen unveränderliches System darstellt, sondern durch die Membran des Membranpumpenantriebs an weitere Systemteile angekoppelt ist. Dadurch, dass die Membran des Membranpumpenantriebs sich erfindungsgemäß während der Bestimmung des Systemkompressibilitätswertes an einer starren Fläche abstützt, kann die Systemkompressibilität des Membranpumpenantriebs jedoch von externen Einflüssen weitgehend abgeschottet werden. Der erfindungsgemäß bestimmte Systemkompressibilitätswert gibt daher die auf den Membranpumpenantrieb selbst zurückgehende Kompressibilität genauer wieder. Weiterhin wird die erfindungsgemäße Bestimmung nicht mehr durch den Gegendruck der Membran des Membranpumpenantriebs beeinflusst.

Der erfindungsgemäße Systemkompressibilitätswert kann dabei ein beliebiger Parameter sein, durch welchen eine Kompressibilitäts-Eigenschaft bzw. die Nachgiebigkeit des Membranpumpenantriebs bei Druckwechseln charakterisiert und bevorzugt quantifiziert werden kann. Als Betriebsparameterwerte, aus welchen der Systemkompressibilitätswert berechnet wird, werden dabei bevorzugt die dem ersten und zweiten Druckniveau zugeordneten Membranpumpenantriebspositionen herangezogen.

Zum Anfahren des ersten und zweiten Druckniveaus wird bevorzugt der Membranpumpenantrieb betätigt, bis der Druck der Membranpumpe und / oder des Membranpumpenantriebs das erste Druckniveau erreicht. Daraufhin wird der erste Betriebsparameterwert des Membranpumpenantriebs bestimmt. Dann wird der Membranpumpenantrieb betätigt, bis der Druck der Membranpumpe und / oder des Membranpumpenantriebs das zweite Druckniveau erreicht und dann der zweite Betriebsparameterwert bestimmt. Der Druck des Membranpumpenantriebs und / oder der Membranpumpe kann dabei über einen Drucksensor erfasst werden. Der Betriebsparameter kann über einen entsprechenden Betriebsparametersensor, beispielsweise einen Positions- und / oder Bewegungssensor bestimmt werden.

Bei dem ersten und dem zweiten Druckniveau kann es sich dabei um vorgegebene Druckniveaus handeln. Insbesondere können diese in einer Steuerung des Membranpumpenantriebs abgelegt sein.

Gemäß einer bevorzugten Ausführungsform der vorliegenden Erfindung übersteigen das erste und zweite Druckniveau, bei welchem die Betriebsparameterwerte bestimmt werden, den maximalen Gegendruck der Membran des Membranpumpenantriebs. Dies sorgt dafür, dass je nachdem, ob mit Unter- oder Überdruck gearbeitet wird, die Membran entweder nach außen oder nach innen maximal ausgelenkt ist, während der beim ersten und zweiten Druckniveau vorliegende jeweilige Betriebsparameterwert bestimmt wird.

Das erfindungsgemäße Verfahren kommt bevorzugt bei solchen Systemen zum Einsatz, bei welchen eine Pumpkassette mit einer darin angeordneten Pumpkammer an den Membranpumpenantrieb ankoppelbar ist.

Gemäß einer ersten Variante der vorliegenden Erfindung kann die Bestimmung des Systemkompressibilitätswertes dabei erfolgen, bevor die Pumpkassette angekoppelt wurde. Bevorzugt liegt in diesem Fall die Membran während der Bestimmung des Systemkompressibilitätswertes an einer Aufnahmefläche einer Pumpkassettenaufnahme an. Eine solche Pumpkassettenaufnahme wird dabei im normalen Pumpbetrieb dazu eingesetzt, um die Pumpkassette an der Ankoppelfläche des Membranpumpenantriebs zu halten. Hierfür weist sie eine Aufnahmefläche auf, an welcher sich im eingelegten Zustand der Pumpkassette die Rückwand der Pumpkammer abstützt. Gemäß der vorliegenden Erfindung kann nun die Bestimmung des Systemkompressibilitätswertes erfolgen, bevor die Pumpkassette eingelegt wurde, wobei die Membran dabei an dieser Aufnahmefläche anliegt. Die Aufnahmefläche kann dabei beispielsweise in etwa der Form der Pumpkammer folgen, und weist üblicherweise eine konkave Form auf.

In einer zweiten Variante der vorliegenden Erfindung kann die Bestimmung des Systemkompressibilitätswertes mit angekoppelter Pumpkassette erfolgen. Dabei kann bei der Bestimmung des Systemkompressibilitätswertes die Membran des Membranpumpenantriebs komplett in die Pumpkammer der Pumpkassette hineingedrückt sein und sich an der Rückwand der Pumpkammer abstützen. Diie Membran des Membranpumpenantriebs liegt in diesem Zustand an der Membran der Pumpkassette an, welche wiederum an der Rückwand der Pumpkammer anliegt.

Bevorzugt erfolgt bei den oben beschriebenen beiden Varianten die Bestimmung des Systemkompressibilitätswertes bei Überdruck. Hierdurch wird die Membran des Membranpumpenantriebs aus einer Antriebskammer heraus gegen die entsprechende Gegenfläche gedrückt.

Die Bestimmung des Systemkompressibilitätswertes kann jedoch auch durch Anlegen eines Unterdrucks erfolgen. Bevorzugt wird die Membran dabei komplett in die Antriebskammer des Membranpumpenantriebs hineingezogen und liegt an einer Rückwand dieser Antriebskammer an. Auch hierdurch kann der Einfluss weiterer Komponenten auf die Bestimmung der Systemkompressibilität des Membranpumpenantriebs verringert bzw. vermieden werden.

Die vorliegende Erfindung kann insbesondere bei solchen Membranpumpenantrieben zum Einsatz kommen, bei welchen im Normalbetrieb die Pumpkassette durch das Bedrucken eines hinter der Pumpkassette angeordneten Luftkissens an eine Ankoppelfläche des Membranpumpenantriebs angepresst wird. Insbesondere kann dabei durch das Befüllen des Luftkissens eine Pumpkassettenaufnahme, in welche die Pumpkassette im Normalbetrieb eingelegt ist, zusammen mit der Pumpkassette gegen die Ankoppelfläche des Membranpumpenantriebes bewegt werden.

Bevorzugt erfolgt dabei die Bestimmung des Systemkompressibilitätswertes, nachdem das Luftkissen auf Betriebsdruck gefüllt wurde. Dies ist unabhängig davon von Vorteil, ob der Systemkompressibilitätswert mit eingelegter Pumpkassette oder ohne eingelegte Pumpkassette bestimmt wird, da durch das Füllen des Luftkissens sichergestellt wird, dass sich die Gegenfläche, an welche sich die Membran anlegt, nicht bewegt. Insbesondere werden hierdurch ein ohne die Anpressung vorliegendes mechanisches Spiel und eine mechanische Verformung bei der Messung verringert oder vermieden. Bevorzugt ist der Betriebsdruck des Luftkissens dabei größer als das erste und zweite Druckniveau, und / oder die durch das Luftkissen auf die Pumpkassettenaufnahme ausgeübte Kraft größer als die durch das erste und zweite Druckniveau über die Membran des Membranpumpenantriebs ausgeübte Kraft, sodass die genaue Höhe des Betriebsdruckes keinen Einfluss auf die Bestimmung der Systemkompressibilität hat.

Die Bestimmung der Systemkompressibilität bei Unterdruck kann dabei sowohl mit eingelegter Pumpkassette, als auch vor Einlegen der Pumpkassette erfolgen.

Erfolgt die Bestimmung des Systemkompressibilitätswertes bei eingelegter Kassette, so ist bevorzugt mindestens ein Ventil der Pumpkammer geöffnet, um eine Fluidströmung in die Pumpkammer bzw. aus der Pumpkammer heraus zu ermöglichen, wenn die Membran sich beim Anfahren des ersten Betriebsdrucks an die Rückwand der Antriebskammer bzw. der Pumpkammer anlegt.

Das erfindungsgemäß eingesetzte erste Druckniveau ist bevorzugt größer als 50 mBar, da die üblicherweise eingesetzten Membranen üblicherweise einen Gegendruck von etwa 50 mBar erzeugen. Bevorzugt ist das erste Druckniveau größer als 75 mBar, weiterhin bevorzugt größer als 100 mBar und weiterhin bevorzugt größer als 150 mBar. Hierdurch wird sichergestellt, dass sich die Membran beim Erreichen des ersten Druckniveaus komplett an die Gegenfläche angelegt hat. Bevorzugt ist das erste Druckniveau jedoch kleiner als 600 mBar. Hierdurch wird sichergestellt, dass der Systemkompressibilitätswert in einem Druckbereich gemessen wird, welcher auch im normalen Pumpbetrieb der Pumpe vorkommt. Bevorzugt ist das erste Druckniveau dabei kleiner als 400 mBar, weiter bevorzugt kleiner als 300 mBar, und weiter bevorzugt kleiner als 250 mBar. Hierdurch wird noch ein gewisser Druckwertebereich oberhalb des ersten Druckniveaus zum Anfahren des zweiten Druckniveaus zur Verfügung gestellt.

Bevorzugt ist das zweite Druckniveau ebenfalls größer als 50 mBar, bevorzugt größer als 100 mBar, weiter bevorzugt größer als 200 mBar, weiter bevorzugt größer als 250 mBar. Hierdurch wird erreicht, dass sich die Membran komplett anlegt. Bevorzugt ist das zweite Druckniveau dabei höher als das erste Druckniveau. Bevorzugt ist das zweite Druckniveau jedoch kleiner als 600 mBar, bevorzugt kleiner als 500 mBar, weiter bevorzugt kleiner als 450 mBar, weiterhin bevorzugt kleiner als 400 mBar. Hierdurch wird der Messbereich für die Bestimmung des Systemkompressibilitätswertes in einem Druckbereich gehalten, welcher auch im normalen Pumpbetrieb erreicht wird.

Die oben angegebenen Druckwerte sind dabei als Absolutwerte der Druckdifferenz über die Membran angegeben, d. h. der Druckdifferenz zwischen dem Druck in der Antriebskammer und dem Druck, welcher an der Außenfläche der Membran anliegt. Es kann sich also um Über- oder Unterdruck in der angegebenen Höhe handeln. Bevorzugt liegt dabei atomsphärischer Druck an der Außenfläche der Membran an, während der Systemkompressibilitätswert bestimmt wird.

Weiter bevorzugt ist die Differenz zwischen dem ersten und dem zweiten Druckniveau größer als 5 mBar, weiter bevorzugt größer als 10 mBar, weiter bevorzugt größer als 40 mBar, weiter bevorzugt größer als 80 mBar. Durch eine ausreichend große Druckdifferenz zwischen dem ersten und dem zweiten Druckniveau wird eine gewisse Genauigkeit bei der Bestimmung des Systemkompressibilitätswertes sichergestellt. Wird die Druckdifferenz dagegen zu klein gewählt, entspricht sie nur einer minimalen Änderung der Pumpenantriebsposition, was den Einfluss von Messfehlern vergrößert.

Bevorzugt ist die Differenz zwischen dem ersten und dem zweiten Druckniveau jedoch kleiner als 500 mBar, bevorzugt kleiner als 400 mBar, weiter bevorzugt kleiner als 300 mBar, weiter bevorzugt kleiner als 200 mBar. Hierdurch können Druckniveaus gewählt werden, welche im normalen Betriebsbereich des Pumpenantriebs während des Pumpens liegen. Weiterhin kann die Messung schnell erfolgen.

Beispielsweise kann das erste Druckniveau ca. 200 mBar und das zweite Druckniveau ca. 300 mBar betragen.

Gemäß dem erfindungsgemäßen Verfahren kann vorgesehen sein, dass sowohl bei Unter- als auch bei Überdruck ein Systemkompressibilitätswert bestimmt wird.

Hierdurch können Effekte, welche sich bei Unter- bzw. Überdruck in unterschiedlicher Weise auf den Systemkompressibilitätswert auswirken, berücksichtigt werden.

Wie bereits oben dargelegt, kann als Systemkompressibilitätswert ein beliebiger Parameter bestimmt werden, welcher mit der Nachgiebigkeit des Pumpenantriebs bei Druckwechsel korreliert ist. Besonders bevorzugt hängt der Systemkompressibilitätswert dabei von der Differenz der Betriebsparameterwerte, welche bei dem ersten und zweiten Druckniveau bestimmt werden, ab. Insbesondere wird der Systemkompressibilitätswert dabei unter Heranziehung dieser Differenz bestimmt.

Bevorzugt hängt der Systemkompressibilitätswert dabei von der Differenz zwischen den Pumppositionen, welche der Membranpumpenantrieb beim ersten und zweiten Druckniveau einnimmt, ab. Insbesondere kann der Systemkompressibilitätswert schlicht als diese Differenz bestimmt werden.

Das erfindungsgemäße Verfahren kommt bevorzugt bei einem Membranpumpenantrieb zum Einsatz, welcher eine Antriebskammer aufweist, welche durch die Membran abgeschlossen ist, wobei die Membran durch Überdruck in der Antriebskammer nach außen aus der Antriebskammer heraus und durch Unterdruck in der Antriebskammer nach innen in die Antriebskammer hinein ausgelenkt wird.

Weiterhin kann der Membranpumpenantrieb einen Drucksensor aufweisen, welcher den Druck in der Antriebskammer bestimmt, um das erste und das zweite Druckniveau anzufahren.

Weiterhin kann der Druck in der Antriebskammer bevorzugt über eine mit der Antriebskammer in Verbindung stehende Kolben-Zylinder-Einheit erzeugt werden. Insbesondere kann die Kolben-Zylinder-Einheit dabei mit der Antriebskammer fluidisch in Verbindung stehen, beispielsweise über einen Verbindungsschlauch. Weiter bevorzugt ist ein Längensensor vorgesehen, welcher die Position des Kolbens als Betriebsparameterwert erfasst.

Bevorzugt erfolgt die Übertragung des Drucks auf die Membran dabei hydraulisch. Insbesondere können die Kolben-Zylinder-Einheit und die Antriebskammer hydraulisch miteinander verbunden sein.

Wie bereits oben dargelegt, sind bei einer solchen Ausführung des Membranpumpenantriebs als Kolbenmembranpumpe die Haupteinflussfaktoren für die Systemkompressibilität die Luft, welche sich in dem Hydraulikfluid des Hydrauliksystems angesammelt hat, sowie die Nachgiebigkeit des Verbindungsschlauches zwischen der Kolben-Zylinder-Einheit und der Antriebskammer. Diese Systemkompressibilität kann nun erfindungsgemäß bestimmt werden, ohne dass über die Membran externe Einflüsse den Messwert verfälschen würden.

Die vorliegende Erfindung umfasst weiterhin einen Membranpumpenantrieb mit einem Drucksensor und mit einer Steuerung, wobei die Steuerung eine Funktion zur Durchführung eines erfindungsgemäßen Verfahrens aufweist, wie es oben beschrieben wurde. Insbesondere kann die Funktion dabei automatisch den Systemkompressibilitätswert des Membranpumpenantriebs bestimmen. Die Steuerung kann hierfür das erste und zweite Druckniveau anfahren und den zugehörigen ersten und zweiten Membranpumpenantriebswert erfassen, wobei die Druckniveaus so gewählt sind, dass sich die Membran auf einer starren Fläche abstützt.

Insbesondere erfolgt die Bestimmung des Systemkompressibilitätswertes dabei in der Aufrüstphase, d. h. vor dem eigentlichen Pumpbetrieb. Die erfindungsgemäße Funktion kann dabei in den Aufrüst-Ablauf integriert sein. Insbesondere kann die erfindungsgemäße Funktion die Bestimmung des Systemkompressibilitätswertes dabei als Teil des Aufrüstprozesses automatisiert und / oder auf eine Benutzereingabe hin durchführen.

Bevorzugt weist der Membranpumpenantrieb weiterhin einen Sensor zur Bestimmung eines Betriebsparameterwertes auf, insbesondere einen Positionssensor zum Bestimmen einer Membranpumpenantriebsposition.

Weiterhin bevorzugt weist der Membranpumpenantrieb bevorzugt eine Ankoppelfläche auf, an welche eine Pumpkassette ankoppelbar ist.

Bevorzugt ist der Membranpumpenantrieb dabei so aufgebaut, wie dies bereits oben im Hinblick auf das erfindungsgemäße Verfahren dargestellt wurde. Insbesondere handelt es sich um den Antrieb einer Kolbenmembranpumpe. Weiterhin führt die Funktion das erfindungsgemäße Verfahren bevorzugt so durch, wie dies bereits oben dargestellt wurde.

Die vorliegende Erfindung umfasst weiterhin, eine Blutbehandlungsmaschine, insbesondere eine Dialysemaschine, insbesondere eine Peritonealdialysemaschine, mit einem solchen Membranpumpenantrieb. Insbesondere weist die Blutbehandlungsmaschine dabei eine Pumpkassettenaufnahme und / oder ein Luftkissen zum Anpressen der Pumpkassette an eine Ankoppelfläche des Membranpumpenantriebs auf. Bevorzugt ist die Steuerung des Membranpumpenantriebs dabei in die Steuerung der Blutbehandlungsmaschine integriert, so dass diese eine Funktion zur Durchführung des erfindungsgemäßen Verfahrens aufweist.

Gemäß einem zweiten Aspekt umfasst die vorliegende Erfindung ein Verfahren zur Bestimmung eines Luftanteils und / oder eine Luftmenge in einer von einer Membranpumpe geförderten medizinischen Flüssigkeit. Hierfür werden durch entsprechendes Ansteuern eines Membranpumpenantriebs der Membranpumpe ein erstes und ein zweites Druckniveau angefahren und zugehörige Betriebsparameterwerte der Membranpumpe erfasst, wobei der Luftanteil und / oder die Luftmenge auf Grundlage der Betriebsparameterwerte bestimmt wird. Anders als gemäß den aus den Druckschriften DE 19919572 A1 und DE 102011105824 B3 bekannten Verfahren wird damit als Ausgangsdruck nicht das sich durch das gravimetrische Befüllen der Pumpkammer zufällig ergebende Druckniveau als erstes Druckniveau eingesetzt, sondern ein vorgegebenes, durch entsprechendes Ansteuern des Membranpumpenantriebs angefahrenes Druckniveau. Hierdurch wird die Bestimmung des Luftanteils bzw. der Luftmenge unabhängig von dem zufällig sich einstellenden Druck in der Druckkammer beim gravimetrischen Befüllen.

Bevorzugt erfolgt die Bestimmung des Luftanteils und / oder der Luftmenge dadurch, dass nach dem Füllen der Pumpkammer sämtliche Ventile der Pumpkammer geschlossen werden, sodass sich ein abgeschlossenes Fluidvolumen innerhalb der Pumpkammer ergibt. Daraufhin wird durch entsprechendes Ansteuern des Membranpumpenantriebs zunächst das erste Druckniveau angefahren und der erste Betriebsparameterwert bestimmt, und dann durch erneutes Ansteuern des Membranpumpenantriebs das zweite Druckniveau angefahren und der zugehörige zweite Betriebsparameterwert bestimmt. Der Luftanteil und / oder die Luftmenge können dabei bevorzugt anhand des ersten und des zweiten Druckniveaus sowie des ersten und des zweiten Betriebsparameterwertes bestimmt werden.

Bevorzugt wird als Betriebsparameterwert dabei eine Pumpenposition bestimmt. Insbesondere kann hierzu bei einer Kolbenmembranpumpe die Position des Kolbens der Kolben-Zylinder-Einheit als Betriebsparameterwert bestimmt werden.

Die Berechnung des Luftanteils und / oder der Luftmenge kann dann gemäß der bereits aus der DE 19919572 A1 bekannten Formel erfolgen.

Bevorzugt kommt das erfindungsgemäße Verfahren dabei bei einer Membranpumpe zum Einsatz, wie sie bereits oben im Hinblick auf den ersten Aspekt näher beschrieben wurde. Insbesondere kann es sich um eine Kolbenmembranpumpe handeln.

Erfindungsgemäß wird bei der Bestimmung des Luftanteils und / oder der Luftmenge gemäß dem zweiten Aspekt ein Systemkompressibilitätswert des Membranpumpenantriebs berücksichtigt. Hierdurch wird die Genauigkeit bei der Bestimmung des Luftanteils und / oder der Luftmenge erhöht.

Bevorzugt werden dabei zur Bestimmung des Systemkompressibilitätswertes ein drittes und ein viertes Druckniveau angefahren und zugehörige Betriebsparameterwerte der Pumpe erfasst, wobei der Systemkompressibilitätswert auf Grundlage der Betriebsparameterwerte bestimmt wird. Bei den Betriebsparameterwerten kann es sich wiederum um Betriebsparameterwerte handeln, welche zur Bestimmung des Luftanteils und / oder der Luftmenge herangezogen werden.

Bevorzugt erfolgt die Bestimmung des Systemkompressibilitätswertes dabei in der Aufrüstphase der Membranpumpe.

Bevorzugt werden bei der Bestimmung des Systemkompressibilitätswertes die beiden gleichen Druckniveaus eingesetzt, welche auch bei der Bestimmung des Luftanteils und / oder Luftmenge eingesetzt werden. Dies hat den großen Vorteil, dass der Systemkompressibilitätswert nicht für eine Vielzahl von Druckniveaus oder Druckänderungen bestimmt werden muss, sondern lediglich für das erste und zweite Druckniveau, und dennoch den Anteil der Systemkompressibilität an den bei der Bestimmung des Luftanteils und / oder Luftmenge gemessenen Werten korrekt wiedergibt. Dieses Vorgehen wird dabei erst durch das erfindungsgemäße Verfahrens zur Bestimmung des Luftanteils und / oder Luftmenge gemäß dem zweiten Aspekt der vorliegenden Erfindung ermöglicht, wonach das erste und das zweite Druckniveau aktiv angefahren werden, sodass hier zwei vorbestimmte Druckniveaus eingesetzt werden können, welche auch bei der Bestimmung des Systemkompressibilitätswertes zum Einsatz kommen.

Zur Bestimmung des Systemkompressibilitätswertes, welcher im Rahmen der Bestimmung des Luftanteils und / oder der Luftmenge gemäß dem zweiten Aspekt berücksichtigt wird, kommt das erfindungsgemäße Verfahren zur Bestimmung eines Systemkompressibilitätswertes gemäß dem ersten Aspekt der Erfindung zum Einsatz.

Die vorliegende Erfindung umfasst weiterhin einen Membranpumpenantrieb mit einem Drucksensor und mit einer Steuerung, wobei die Steuerung eine Funktion zur Durchführung eines erfindungsgemäßen Verfahrens zur Bestimmung eines Luftanteils und / oder einer Luftmenge gemäß dem zweiten Aspekt der vorliegenden Erfindung aufweist. Bevorzugt führt die Steuerung das erfindungsgemäße Verfahren dabei automatisiert durch, insbesondere während des laufenden Pumpbetriebs. Insbesondere kann der hierdurch bestimmte Luftanteil und / oder die hierdurch bestimmte Luftmenge dabei bei der Bilanzierung der durch die Membranpumpe geförderten Flüssigkeit berücksichtigt werden.

Bevorzugt weist der Membranpumpenantrieb eine Ankoppelfläche auf, an welcher eine Pumpkassette ankoppelbar ist. Weiterhin bevorzugt weist der Membranpumpenantrieb einen Sensor zur Bestimmung eines Betriebsparameterwertes auf, insbesondere einen Sensor zur Bestimmung einer Pumpenantriebsposition.

Bevorzugt ist der erfindungsgemäße Membranpumpenantrieb dabei so aufgebaut, wie dies bereits oben im Hinblick auf die erfindungsgemäßen Verfahren gemäß dem ersten Aspekt der vorliegenden Erfindung dargestellt wurde.

Besonders bevorzugt weist die Steuerung des erfindungsgemäßen Membranpumpenantriebs dabei sowohl eine Funktion zur Durchführung eines Verfahrens zur Bestimmung eines Systemkompressibilitätswertes gemäß dem ersten Aspekt, als auch eine Funktion zur Bestimmung eines Luftanteils und / oder einer Luftmenge in der von dem Membranpumpenantrieb geförderten medizinischen Flüssigkeit gemäß dem zweiten Aspekt der vorliegenden Erfindung auf.

Die vorliegende Erfindung umfasst weiterhin eine Blutbehandlungsmaschine, insbesondere eine Dialysemaschine, insbesondere eine Peritonealdialysemaschine, mit einem Membranpumpenantrieb gemäß dem ersten und/oder zweiten Aspekt.

Die Blutbehandlungsmaschine weist bevorzugt eine Pumpkassettenaufnahme und / oder Luftkissen zum Anpressen der Pumpkassette an eine Ankoppelfläche des Membranpumpenantriebs auf.

Bevorzugte Ausgestaltungen der vorliegenden Erfindung werden nun anhand von Figuren und Ausführungsbeispielen näher dargestellt:

Dabei zeigen:

Fig. 1: eine schematische Darstellung eines erfindungsgemäßen Membranpumpenantriebs mit angekoppelter Pumpenkammer,

Fig. 2: einen Schnitt durch den Ankopplungsbereich eines erfindungsgemäßen Membranpumpenantriebs mit angekoppelter Pumpkassette, und

Fig. 3: ein Ausführungsbeispiel einer Pumpkassette, wie sie an einen erfindungsgemäßen Membranpumpenantrieb angekoppelt werden kann.

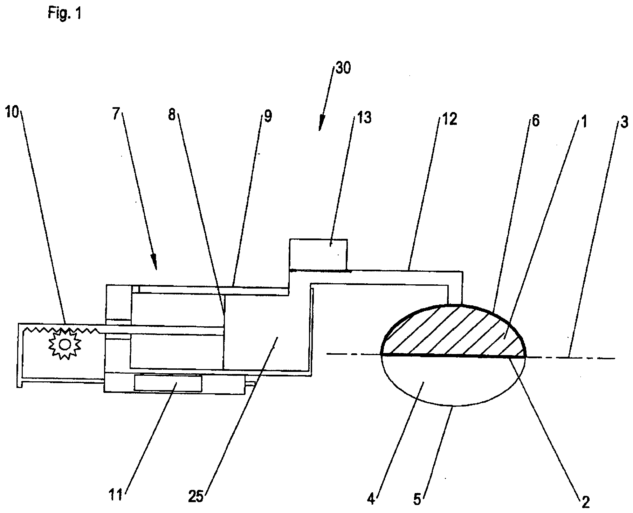

Fig. 1 zeigt ein Ausführungsbeispiel eines erfindungsgemäßen Membranpumpenantriebs 30 zum Pumpen einer medizinischen Flüssigkeit durch die Pumpkammer 4, welche an den Membranpumpenantrieb ankoppelbar ist.

Der Membranpumpenantrieb weist eine Antriebskammer 1 auf, an welcher eine flexible Membran 2 angeordnet ist. Die flexible Membran 2 ist in einer Ankoppelfläche 3 des Membranpumpenantriebs angeordnet, so dass eine in Fig. 1 nicht erkennbare Membran der Pumpkammer 4 an die Membran 2 der Antriebskammer so angekoppelt werden kann, dass diese den Bewegungen der Membran 2 der Antriebskammer folgt. Durch eine Bewegung der Membran 2 aus der Antriebskammer 1 heraus bzw. in diese hinein kann daher das Volumen der Pumpkammer 4 verändert werden. Durch das entsprechende Schalten von in Fig. 1 nicht näher dargestellten Ventilen, welche den Zu- bzw. Abfluss zur Pumpkammer 4 steuern, kann durch Bewegen der Membran 2 mit der Pumpkammer 4 Fluid gepumpt werden.

Die Pumpkammer 4 ist dabei üblicherweise Teil einer in Fig. 1 nicht näher dargestellten Pumpkassette, welche bevorzugt ein Disposable darstellt. Dabei wird die Pumpkammer üblicherweise durch eine entsprechende Ausformung eines Hartteils der Pumpkassette gebildet, welche durch eine die Membran der Pumpkammer bildende flexible Folie abgedeckt wird.

Die vorliegende Erfindung wäre jedoch in gleicher Weise auch bei Membranpumpen einsetzbar, bei welchen die Antriebskammer und die Pumpkammer fest miteinander verbunden bzw. in einer gemeinsamen Pumpvorrichtung integriert sind.

Bei dem in Fig. 1 gezeigten Ausführungsbeispiel handelt es sich dabei um eine Kolbenmembranpumpe, welche eine Kolben-Zylinder-Einheit 7 aufweist, die über die Hydraulikleitung 12 mit der Antriebskammer 6 hydraulisch in Verbindung steht. Die Kolben-Zylinder-Einheit 7 wird dabei von einem Antrieb 10 angetrieben, welcher auf den Kolben 8 der Kolben-Zylinder-Einheit 7 einwirkt und diesen im Zylinder 9 bewegt. Die Wegstrecke, die der Kolben 8 im Zylinder 9 verfahren wird, wird von einem der Kolben-Zylinder-Einheit 7 zugeordneten Längensensor 11 erfasst bzw. gemessen.

Die Druckseite 25 der Kolben-Zylinder-Einheit 7 steht dabei über die Fluidleitung 12 fluidisch mit der Antriebskammer 1 in Verbindung, wobei die Druckseite 25, die Fluidleitung 12 und die Antriebskammer 1 mit Hydraulikfluid gefüllt sind. Hierdurch wird die Stellbewegung des Kolbens 8 auf die Membran 2 der Antriebskammer 1 übertragen. Die Membran 2 der Antriebskammer 1 wird daher bei entsprechender Veränderung des Hydraulikvolumens der Kolben-Zylinder-Einheit 7 durch Bewegung des Kolbens 8 konvex nach außen gewölbt bzw. konkav in den Innenraum der Antriebskammer hineingezogen.

Die zur Fluidförderung in der Pumpkammer 4 erforderliche Volumenveränderung der Antriebskammer 1 wird dementsprechend durch Betätigung der Kolben-Zylinder-Einheit 7 herbeigeführt. Durch Betätigung des Kolbens 8 wird das Hydraulikfluid in die Antriebskammer 1 gedrückt bzw. aus dieser abgesaugt. Hierdurch wird die Membran 2 betätigt, deren Bewegung auf die Pumpkammer 5 übertragen wird und diese in ihrem Volumen verändert.

Der Membranpumpenantrieb weist weiterhin einen Drucksensor 13 auf, über welchen der Druck des Hydraulikfluides im Hydrauliksystem, und damit der Druck in der Antriebskammer 1 gemessen werden kann. Der in der Antriebskammer 1 herrschende Druck entspricht dabei - bis auf einen etwaigen Gegendruck der Membran 2 - dem in der Pumpkammer 4 herrschenden Gegendruck, so dass über den Drucksensor 13 gleichzeitig auch der Druck in der Pumpkammer 4 bestimmt werden kann.

Der Membranpumpenantrieb weist weiterhin eine nicht dargestellte Steuerung auf, welche mit dem Längensensor 11 und dem Drucksensor 13 in Verbindung steht und die Messsignale auswertet. Weiterhin steuert die Steuerung den Antrieb 10 des Membranpumpenantriebs sowie die Ventile zur Steuerung des Fluidstroms in und aus der Pumpkammer 4 an.

Eine solche Kolbenmembranpumpe besitzt den Vorteil, dass sie Flüssigkeit sehr mengengenau fördert, wobei die insgesamt geförderte Menge präzise bilanziert werden kann, da das Pumpvolumen dem Hubvolumen der Kolben-Zylinder-Einheit 7 entspricht und durch den Längensensor 11 exakt messbar ist.

Die Steuerung des Membranpumpenantriebs der vorliegenden Erfindung weist dabei zunächst eine Funktion gemäß dem zweiten Aspekt der vorliegenden Erfindung auf, durch welche ein Luftanteil und / oder eine Luftmenge in der von der Membranpumpe geförderten Flüssigkeit bestimmt werden kann. Durch diese Funktion kann verhindert werden, dass Luftblasen, welche in der Pumpkammer 4 vorhanden sind, die Bilanzierung der durch die Pumpkammer 4 geförderten Flüssigkeit verfälschen.

Zur Bestimmung des Luftanteils bzw. der Luftmenge ist eine Messphase vorgesehen, welche dem Pumpvorgang bei jedem Hub zwischengeschaltet werden kann.

Zunächst wird dabei gemäß dem üblichen Pumpvorgang durch Bewegung der Membran 2 Fluid in die Pumpkammer 4 eingesaugt. Daraufhin werden die Absperrventile der Pumpkammer 4 geschlossen, so dass sich ein abgeschlossenes Fluidvolumen ergibt, und durch Betätigen des Antriebs 10 ein erstes, vorbestimmtes Druckniveau $p_a$ angefahren und die zugehörige Position des Kolbens 8 bestimmt. Daraufhin wird wiederum durch Betätigung des Antriebs 10 ein zweites Druckniveau $p_e$ angefahren, und ebenfalls die zugehörige Position des Kolbens 8 bestimmt. Weist die in der Pumpkammer 4 eingeschlossene Flüssigkeit einen gewissen Gasanteil auf, wird dieser durch die Druckerhöhung komprimiert, was einer entsprechenden Veränderung des Volumens der Pumpkammer 4 entspricht. Diese Volumendifferenz kann durch die beim Anfangs- und beim Enddruck vorliegenden Positionen des Kolbens 9 bestimmt werden.

Aus den so gewonnen Werten berechnet die Steuerung die in der Pumpkammer enthaltene Luftmenge, d. h. das dort enthaltene Luftvolumen $V_{at}$ bei atmosphärischem Druck. Hierzu geht die Steuerung von dem Boyle-Mariotte-Gesetz aus, welches für eine isothermische Zustandsänderung, d. h. bei Vernachlässigung einer Temperaturänderung, lautet:

$$p \times V = konstant.$$

Hiervon ausgehend können verschiedene Zustände der Messphase gleichgesetzt werden:

$$V_{at} \times p_{at} = V_a \times p_a = V_e \times p_e.$$

Unter Beachtung des Zusammenhangs, dass das Differenzvolumen $V_{diff}$ durch die Differenz des Ausgangsvolumens und des Endvolumens bestimmt ist, also $V_{diff} = V_a - V_e$, kann hieraus das tatsächliche Gasvolumen bei atmosphärischem Druck $V_{at}$ gewonnen werden:

$$V_{at} = \frac{V_{diff}}{\left( \dfrac{p_{at}}{p_a} - \dfrac{p_{at}}{p_a + p_{diff}} \right)}$$

Je nach dem konkret eingesetzten Pumpverfahren muss bei dieser Formel berücksichtigt werden, dass der auf der Hydraulikseite der Membranpumpe über den Drucksensor 13 gemessene Druck ggf. nicht exakt dem Druck in der Pumpkammer 4 entspricht, sondern durch die Eigenspannung der Membran 2 um einen gewissen Wert von diesem Druck abweicht. In einer ersten Variante des Verfahrens kann die Bestimmung des Luftanteils jedoch mit nicht ausgelenkter Membran 2 erfolgen, so dass der Einfluss der Membran vernachlässigbar ist. In einer zweiten Variante kann der Ausgangsdruck $p_a$ dagegen um einen auf die Membran zurückzuführenden Differenzdruck $p_{mem}$ zwischen Hydraulikseite und Pumpseite korrigiert werden. Dieser kann bspw. in der Steuerung abgelegt sein. Hierdurch ist es möglich, die Bestimmung des Luftanteils durchzuführen, während die Membran 2 sehr weit oder komplett in die Antriebskammer 1 hineingezogen ist, so dass das komplette Pumpvolumen ausgenutzt wird. Ggf. kann der auf die Membran zurückzuführenden Differenzdruck $p_{mem}$ zwischen Hydraulikseite und Pumpseite dabei in der Aufrüstphase bestimmt werden. Je nach dem Verhältnis zwischen den Drücken auf der Hydraulikseite und dem auf die Membran zurückzuführenden Differenzdruck $p_{mem}$ und der geforderten Genauigkeit kann der Differenzdruck $p_{mem}$ ggf. aber auch vernachlässigt werden.

Die in die obige Formel eingehende Volumendifferenz wird durch die bei der Kompression von dem Druckniveau $p_a$ zum Druckniveau $p_e$ zurückgelegte Wegstrecke des Kolbens $S_{diff}$ und dessen Fläche $A_K$ bestimmt.

Allerdings ist hierbei zu berücksichtigen, dass die Bewegung des Kolbens 8 bei der Druckänderung von $p_a$ nach $p_e$ nicht ausschließlich auf das Luftvolumen in der Pumpkammer 4 zurückzuführen ist. Denn auch der Membranpumpenantrieb selbst weist unter Druckveränderungen eine gewisse Nachgiebigkeit bzw. Systemkompressibilität auf. Faktoren sind hierbei insbesondere die Luft, welche sich im Hydrauliksystem ansammeln kann, sowie eine gewisse Flexibilität der Hydraulikleitung 12. Daher würde der Kolben 8 bei einer Druckänderung von $p_a$ auf $p_e$ auch dann, wenn überhaupt keine Luft in der Pumpkammer 4 enthalten wäre und diese damit inkompressibel wäre, sich allein aufgrund dieser Systemkompressibilität um eine gewisse Wegstrecke $S_0$ bewegen.

Das tatsächliche Volumen $V_{at}$ der in der Pumpkammer 4 enthaltenen Luft ergibt sich damit unter Berücksichtigung des die Systemkompressibilität charakterisierenden Systemkompressibilitätswertes $S_0$ als:

$$V_{at} = \frac{(S_{diff} - S_0) \cdot A_k}{\left( \dfrac{p_{at}}{p_a} - \dfrac{p_{at}}{p_a + p_{diff}} \right)}$$

Dadurch, dass gemäß dem zweiten Aspekt der vorliegenden Erfindung bei der Bestimmung des Luftvolumens in der Pumpkammer 4 zwei zuvor festgelegte Druckniveaus $p_a$ und $p_e$ aktiv angefahren werden, kann der die Systemkompressibilität charakterisierende Systemkompressibilitätswert $S_0$ exakt für diese Druckänderung bestimmt werden. Ungenauigkeiten, welche sich gemäß dem Stand der Technik ergaben durch die Verwendung des durch gravimetrische Füllung erhaltenen Druckniveaus als Ausgangsdruckniveau $p_a$ ergaben, werden hierdurch vermieden.

Die Steuerung des erfindungsgemäßen Membranpumpenantriebs weist dabei bevorzugt eine zweite Funktion auf, über welche der Systemkompressibilitätswert $S_0$ bestimmt werden kann. Auch hierzu werden, beispielsweise in der Aufrüstphase, das erste und das zweite Druckniveau $p_a$ und $p_e$ angefahren, und die entsprechenden Positionen des Kolbens 8 erfasst. Um hierbei jedoch ausschließlich Effekte zu berücksichtigen, welche auf die Systemkompressibilität des Membranpumpenantriebs zurückgehen, und nicht etwa auf die Kompressibilität der an dem Membranpumpenantrieb angekoppelten Komponenten, erfolgt die Bestimmung in einem Zustand des Membranpumpenantriebs, in welchem sich die Membran 2 an einer starren Fläche abstützt. Dies kann beispielsweise dadurch erreicht werden, dass die Bestimmung des Systemkompressibilitätswertes in einem Druckbereich erfolgt, in welchem die Membran 2 maximal nach außen bzw. nach innen ausgelenkt ist.

Die Bestimmung des Systemkompressibilitätswertes kann dabei sowohl mit einer an die Ankoppelfläche 3 des Membranpumpenantriebs angekoppelten Pumpkassette, als auch ohne angekoppelte Pumpkassette erfolgen.

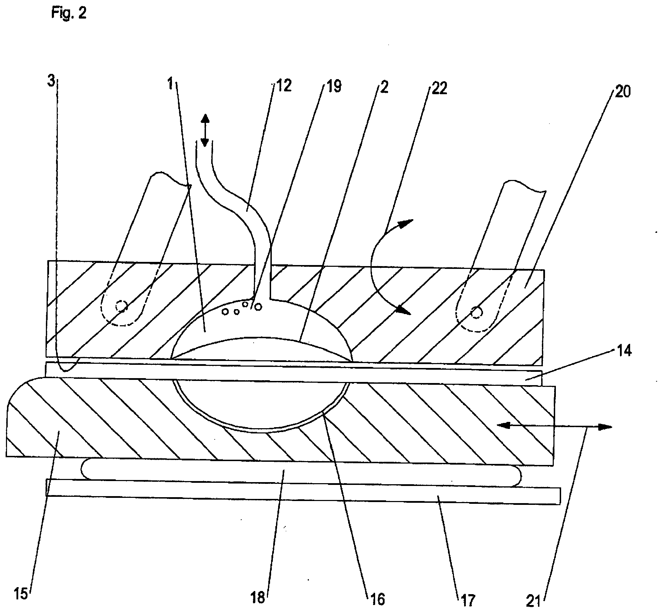

Der mechanische Aufbau eines Ausführungsbeispiels eines erfindungsgemäßen Membranpumpenantriebs, an welchen eine Pumpenkassette angekoppelt werden kann, ist dabei in Fig. 2 näher dargestellt. Der Membranpumpenantrieb weist einen Maschinenblock 20 auf, an welchem die Ankoppelfläche 3 zum Ankoppeln der Pumpkassette 14 angeordnet ist. Die mit der flexiblen Membran 2 versehene Antriebskammer 1 ist dabei in die Ankoppelfläche 3 eingelassen, und steht über die Hydraulikleitung 12 mit der hier nicht näher gezeigten Kolben-Zylinder-Einheit 7 in hydraulischer Verbindung.

Die Pumpkassette 14 wird dabei zum Ankoppeln an die Ankoppelfläche 3 in eine Pumpkassettenaufnahme 15 eingelegt, so dass sich die Rückseite der Pumpkassette an einer Aufnahmefläche der Pumpkassettenaufnahme 15 abstützt. Die Aufnahmefläche weist dabei im Bereich der Pumpkammer 4, welche als eine Auswölbung der Rückseite der Pumpkassette gestaltet ist, eine entsprechende kalottenförmige Ausnehmung auf.

Nach dem Einlegen der Kassette 14 wird die Kassettenaufnahme 15 über ein rückseitig angeordnetes Luftkissen 18, welches sich seinerseits an einer Gerätewand 17 abstützt, gegen die Ankoppelfläche 3 gepresst. Hierzu wird das Luftkissen mit einem entsprechenden Betriebsdruck beaufschlagt, welcher beispielsweise zwischen 1.500 und 2.500 mBar liegen kann.

Im Ausführungsbeispiel ist die Pumpkassettenaufnahme 15 als eine Schublade ausgestaltet, welche in Richtung 21 ein- und ausgefahren werden kann, um eine Kassette einzulegen. Weiterhin ist der Maschinenblock 20 in Bewegungsrichtung 22 auf die Pumpkassette 14 aufsetzbar. Nach dem Einschieben der Schublade 15 und dem Aufsetzen des Maschinenblocks 20 wird dann das Luftkissen 18 bedruckt, um eine sichere Ankopplung der Pumpkassette 14 an die Ankoppelfläche 3 zu erreichen.

Alternativ zu der in Fig. 2 gezeigten konstruktiven Ausgestaltung könnte die Pumpkassettenaufnahme 15 jedoch beispielsweise auch als eine Tür ausgestaltet werden, welche zum Einlegen der Pumpkassette 14 geöffnet und zum Anlegen der Pumpkassette an die Ankoppelfläche geschlossen wird. Das Luftkissen 18 wäre in diesem Fall in die Tür integriert.

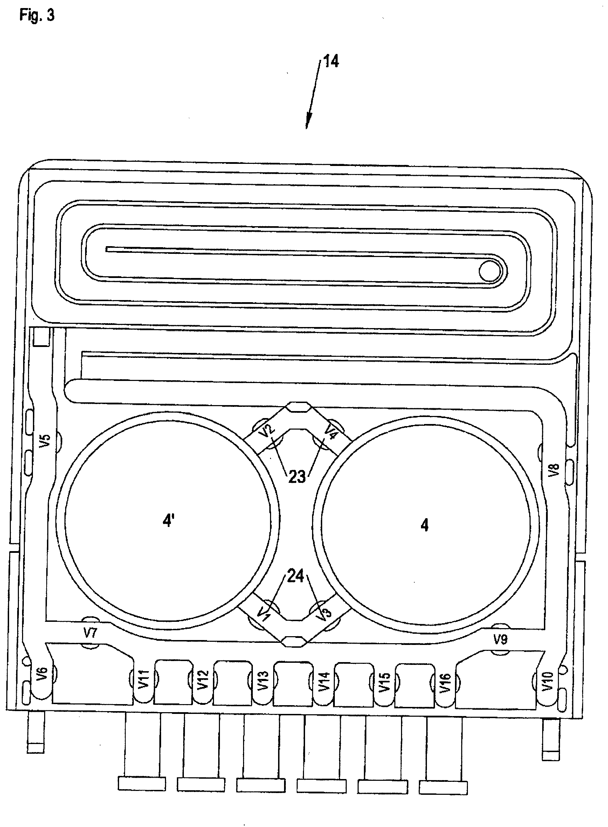

In Fig. 3 ist dabei ein Ausführungsbeispiel einer Pumpkassette 14 gezeigt, welche zwei Pumpkammern 4 und 4' aufweist. Die Pumpkassette besteht dabei aus einem Hartteil, in welchem die flüssigkeitsführenden Kanäle sowie die Pumpkammern eingelassen sind, und wird zur Ankoppelfläche hin durch eine flexible Folie bedeckt. Die Pumpkassette weist dabei u. a. die Ventile 23 und 24 auf, über welche der Fluidstrom in die Pumpkammern 4 und 4' bzw. aus diesen heraus angesteuert werden kann. Die Ventile werden dabei ebenfalls über im Maschinenblock 20 angeordnete Aktoren betätigt.

Die erfindungsgemäße Bestimmung des Systemkompressibilitätswertes erfolgt dabei bevorzugt in der Aufrüstphase der Membranpumpe, kann dabei jedoch sowohl mit eingelegter Pumpkassette, als auch ohne eingelegte Pumpkassette durchgeführt werden.

Wird die Bestimmung durchgeführt, solange keine Pumpkassette 14 eingelegt wurde, so stützt sich die Membran während der Durchführung der Messungen auf der Aufnahmefläche 16 der Pumpkassettenaufnahme 15 ab. Wird die Bestimmung dagegen mit eingelegter Pumpkassette durchgeführt, stützt sich die Membran 2 auf der Rückwand 5 der Pumpkammer 4 ab, und damit auf dem Hartteil der Pumpkassette. Bei eingelegter Pumpkassette muss hierzu mindestens eines der Ventile, welche die Fluidströme in und aus der jeweiligen Pumpkammer ansteuern, geöffnet sein. Vorteilhafterweise erfolgt die Bestimmung des Systemkompressibilitätswertes dabei vor der Füllung der Pumpkassette mit Flüssigkeit, oder während die Pumpkammer über die Fluidverbindungen beispielsweise mit dem Dialysatbeutel oder dem Drainagebeutel in Verbindung steht.

Dass die Membran während der Messphase an der Aufnahmefläche 16 der Pumpkassettenaufnahme bzw. an der Rückwand der Pumpkammer anliegt, wird durch entsprechend hohe Druckniveaus $p_a$ und $p_e$ erreicht, welche für eine komplette Auslenkung der Membran während des Messverfahrens sorgen. Die Spannung der Membran 2 wird dabei bereits durch das Erreichen des ersten Druckniveaus $p_a$ komplett überwunden. Bei der Druckerhöhung auf das zweite Druckniveau $p_e$ stützt sich die Membran dann auf einer starren Gegenfläche ab, so dass die Membran bzw. daran angekoppelte Komponenten keinen Einfluss auf die Bestimmung des Systemkompressibilitätswertes haben.

Da die Eigenspannung der Membran 2 bei einem Druckniveau von ca. 50 mBar überwunden ist, liegt ein geeignetes erstes Druckniveau bei ca. 200 mBar, ein geeignetes zweites Druckniveau bei ca. 300 mBar.

Bevorzugt werden dabei die gleichen Druckniveaus zur Bestimmung des Systemkompressibilitätswertes eingesetzt, welche auch zur Bestimmung des Luftvolumens in der durch die Pumpe geförderten Flüssigkeit herangezogen werden. Als Systemkompressibilitätswert $S_0$ kann hierdurch schlicht die Wegstrecke, welcher der Kolben 8 bei der Druckerhöhung von $p_a$ auf $p_e$ zurücklegt, herangezogen werden. Der Systemkompressibilitätswert $S_0$ ergibt sich damit als die Differenz aus den bei den Druckniveaus $p_a$ und $p_e$ bestimmten Positionswerten des Membranpumpenantriebs.

Wie bereits oben dargestellt, stellen der Luftgehalt der Hydraulikflüssigkeit sowie die Steifigkeit der Hydraulikschläuche die Haupteinflussfaktoren auf die Systemkompressibilität dar. Allerdings können auch mechanische Toleranzen sowie eine Verformung der mechanischen Komponenten zu einer gewissen Nachgiebigkeit des Systems und damit zu einer Erhöhung des Systemkompressibilitätswertes führen.

Die Bestimmung des Systemkompressibilitätswertes erfolgt daher bevorzugt, nachdem das Luftkissen 18 mit Betriebsdruck befüllt wurde, so dass die Pumpkassettenaufnahme 15 gegen die Ankoppelfläche 3 gepresst wird. Hierdurch wird der Einfluss, welchen das Spiel der Pumpkassettenaufnahme sowie eine eventuelle mechanische Verformung der beteiligten mechanischen Komponenten auf die Bestimmung des Systemkompressibilitätswertes haben könnte, verringert. Zudem erfolgt die Bestimmung des Systemkompressibilitätswertes hierdurch in der gleichen Situation, welche dann auch bei der Bestimmung des Luftanteils während des Pumpbetriebs vorliegt. Die Pumpkassettenaufnahme wird dabei unabhängig davon, ob die Bestimmung des Systemkompressibilitätswertes mit oder ohne eingelegte Pumpkassette durchgeführt wird, über das Luftkissen 18 gegen die Ankoppelfläche gepresst.

Soweit der Systemkompressibilitätswert dabei durch den Betriebsdruck des Luftkissens beeinflusst wird, kann zur weiteren Steigerung der Genauigkeit die Regeltoleranz des Luftkisseninnendrucks eingeschränkt werden.

Gemäß den oben beschriebenen Varianten erfolgt die Bestimmung des Systemkompressibilitätswertes bei Überdruck, so dass die Membran 2 sich nach außen aus der Antriebskammer 1 heraus wölbt und sich auf einer außen angeordneten Gegenfläche abstützt.

Die erfindungsgemäße Bestimmung des Systemkompressibilitätswertes lässt sich jedoch auch bei Unterdruckniveaus erreichen. In diesem Fall werden die Unterdruckniveaus so gewählt, dass sich die Membran 2 auf der Rückwand 6 der Antriebskammer 1 abstützt. Ein geeignetes erstes Druckniveau liegt dabei bei ca. -200 mBar, ein geeignetes zweites Druckniveau bei ca. -300 mBar. Die geeigneten Unterdruckniveaus entsprechen damit betragsmäßig den geeigneten Überdruckniveaus.

Bevorzugt wird in dem Fall, dass die Bestimmung des Systemkompressibilitätswertes bei Unterdruckniveaus $p_a$ und $p_e$ erfolgt, auch zur Bestimmung des Luftvolumens in der durch die Pumpkammer gepumpten Flüssigkeit gemäß dem oben dargestellten zweiten Aspekt mit Unterdruckniveaus $p_a$ und $p_e$ gearbeitet werden.

Wird dabei bei der Bestimmung des Systemkompressibilitätswertes mit Unterdruckniveaus gearbeitet, wirken sich die mechanischen Eigenschaften des Aufbaus aus Luftkissen, Pumpkassettenaufnahme und Maschinenblock nicht auf die Messung aus.

Weiterhin kann erfindungsgemäß vorgesehen sein, jeweils einen Systemkompressibilitätswert mit Überdruckniveaus und mit Unterdruckniveaus zu bestimmen. Durch die Anwendung beider Verfahren können die mechanischen Eigenschaften des Aufbaus aus Luftkissen, Pumpkassettenaufnahme und Maschinenblock ermittelt und von den auf das Hydrauliksystem zurückgehenden Eigenschaften separiert werden.

Die Bestimmung des Systemkompressibilitätswertes mit Unterdruck kann ebenfalls mit oder ohne eingelegte Pumpkassette erfolgen. Erfolgt sie mit eingelegter Pumpkassette, so sollten die Ventile, mit welchen die Pumpkammer mit weiteren Komponenten kommunizieren, geöffnet sein.

Der erfindungsgemäß bestimmte Systemkompressibilitätswert kann dabei zum einen wie oben dargestellt in die Bestimmung des Luftvolumens der geförderten medizinischen Flüssigkeit eingehen. Er erlaubt dabei eine genauere Bilanzierung der durch die Membranpumpe bewegten Flüssigkeiten, da das Luftvolumen in den gepumpten Flüssigkeiten genauer bestimmt werden kann.

Die Bestimmung des Systemkompressibilitätswert kann weiterhin zur Verifizierung der Qualität der Entgasung des Hydrauliksystems eingesetzt werden. Beispielsweise kann dabei, sobald der Systemkompressibilitätswert eine gewisse Schwelle übersteigt, eine Entgasung des Hydrauliksystems vorgenommen oder deren Notwendigkeit angezeigt werden.

Der erfindungsgemäße Membranpumpenantrieb wird bevorzugt in einem Blutbehandlungsgerät zum Pumpen medizinischer Flüssigkeiten eingesetzt, insbesondere zum Pumpen von Blut oder Dialysat. Besonders bevorzugt kommt der erfindungsgemäße Membranpumpenantrieb dabei bei einer Dialysemaschine zum Einsatz, wobei die Membranpumpe zum Pumpen des Dialysats in den Bauchraum des Patienten bzw. Abziehen des Dialysats aus dem Bauchraum des Patienten eingesetzt wird.

**Patentansprüche**

1. Verfahren zur Bestimmung eines Systemkompressibilitätswertes eines medizinischen Membranpumpenantriebs, wobei ein erstes und ein zweites Druckniveau angefahren und ein erster und zweiter Betriebsparameterwert des Membranpumpenantriebs erfasst werden, wobei der Systemkompressibilitätswert auf Grundlage der erfassten Betriebsparameterwerte bestimmt wird,
   **dadurch gekennzeichnet,**
   **dass** die Membran des Membranpumpenantriebs sich während der Bestimmung des Systemkompressibilitätswertes an einer starren Fläche abstützt.

2. Verfahren nach Anspruch 1, wobei das erste und zweite Druckniveau den maximalen Gegendruck der Membran übersteigen.

3. Verfahren nach Anspruch 1 oder 2, wobei eine Pumpkassette an den Membranpumpenantrieb ankoppelbar ist, und wobei die Bestimmung des Systemkompressibilitätswertes erfolgt, bevor die Pumpkassette angekoppelt wurde, wobei die Membran bevorzugt an einer Aufnahmefläche einer Pumpkassettenaufnahme anliegt.

4. Verfahren nach Anspruch 1 oder 2, wobei eine Pumpkassette an den Membranpumpenantrieb ankoppelbar ist, und wobei die Bestimmung des Systemkompressibilitätswertes mit angekoppelter Pumpkassette erfolgt, wobei die Membran bevorzugt komplett in eine Pumpkammer der Pumpkassette hineingedrückt ist und sich an der Rückwand der Pumpkammer abstützt.

5. Verfahren nach Anspruch 1 oder 2, wobei die Bestimmung des Systemkompressibilitätswertes bei Unterdruck erfolgt, wobei die Membran bevorzugt komplett in eine Antriebskammer des Membranpumpenantriebs hineingezogen ist und an einer Rückwand der Antriebskammer anliegt.

6. Verfahren nach einem der vorangegangenen Ansprüchen, wobei vor der Bestimmung des Systemkompressibilitätswertes ein Luftkissen, über welches im Normalbetrieb eine Pumpkassette an eine Ankoppelfläche des Membranpumpenantriebs angepresst wird, auf Betriebsdruck gefüllt wird.

7. Verfahren nach einem der vorangegangenen Ansprüchen, wobei das erste Druckniveau größer als 50mBar (50hPa) bevorzugt größer 75mBar, weiter bevorzugt größer 100mBar, weiter bevorzugt größer 150mBar ist, und/oder wobei das erste Druckniveau kleiner als 600mBar (600hPa) bevorzugt kleiner 400mBar, weiter bevorzugt kleiner 300mBar, weiter bevorzugt kleiner 250mBar ist, und/oder wobei das zweite Druckniveau größer als 50mBar (50hPa) bevorzugt größer 100mBar, weiter bevorzugt größer 200mBar, weiter bevorzugt größer 250mBar ist, und/oder wobei das zweite Druckniveau kleiner als 600mBar, bevorzugt kleiner 500mBar, weiter bevorzugt kleiner 450mBar, weiter bevorzugt kleiner 400mBar ist, und/oder wobei die Differenz zwischen dem ersten und dem zweiten Druckniveau größer als 5mBar (5hPa) bevorzugt größer 10mBar, weiter bevorzugt größer 40mBar, weiter bevorzugt größer 80mBar ist, und/oder wobei die Differenz zwischen dem ersten und dem zweiten Druckniveau kleiner als 500mBar (500hPa) bevorzugt kleiner 400mBar, weiter bevorzugt kleiner 300mBar, weiter bevorzugt kleiner 200mBar ist.

8. Verfahren nach einem der vorangegangenen Ansprüchen, wobei sowohl bei Unterdruck als auch bei Überdruck ein Systemkompressibilitätswert bestimmt wird.

9. Verfahren nach einem der vorangegangenen Ansprüchen, wobei der Systemkompressibilitätswert von der Differenz der Betriebsparameterwerte, welche bei dem ersten und zweiten Druckniveau bestimmt werden, abhängt, wobei der Systemkompressibilitätswert bevorzugt von der Differenz zwischen den Pumppositionen, welche der Membranpumpenantrieb beim ersten und zweiten Druckniveau einnimmt, abhängt und weiter bevorzugt dieser Differenz entspricht.

10. Verfahren nach einem der vorangegangenen Ansprüchen, wobei der Membranpumpenantrieb eine Antriebskammer aufweist, welche durch die Membran abgeschlossen ist, wobei die Membran durch Überdruck in der Antriebskammer nach außen aus der Antriebskammer heraus und durch Unterdruck in der Antriebskammer nach innen in die Antriebskammer hinein ausgelenkt wird, und/oder wobei ein Drucksensor vorgesehen ist, welcher den Druck in der Antriebskammer bestimmt, um das erste und das zweite Druckniveau anzufahren, und/oder wobei der Druck in der Antriebskammer bevorzugt über eine mit der Antriebskammer in Verbindung stehende Kolben-Zylinder-Einheit erzeugt wird, wobei bevorzugt ein Längensensor vorgesehen ist, welcher die Position des Kolbens als Betriebsparameterwert erfasst, und/oder wobei die Übertragung des Drucks auf die Membran bevorzugt hydraulisch erfolgt, wobei bevorzugt die Kolben-Zylindereinheit und die Antriebskammer mit Hydraulikfluid gefüllt sind.

11. Verfahren zur Bestimmung eines Luftanteils und/oder einer Luftmenge in einer von einer Membranpumpe geförderten medizinischen Flüssigkeit, wofür durch entsprechendes Ansteuern eines Membranpumpenantriebs der Membranpumpe ein erstes und ein zweites Druckniveau angefahren und zugehörige Betriebsparameterwerte der Membranpumpe erfasst werden, wobei der Luftanteil und/oder die Luftmenge auf Grundlage der Betriebsparameterwerte bestimmt wird, wobei bei der Bestimmung des Luftanteils und/oder der Luftmenge ein Systemkompressibilitätswert des Membranpumpenantriebs berücksichtigt wird, wobei die Bestimmung des Systemkompressibilitätswertes gemäß einem Verfahren nach einem der Ansprüche 1 bis 10 erfolgt.

12. Verfahren nach Anspruch 11, wobei bei der Bestimmung des Luftanteils und/oder der Luftmenge ein Systemkompressibilitätswert des Membranpumpenantriebs berücksichtigt wird, wobei zur Bestimmung des Systemkompressibilitätswertes ein drittes und ein viertes Druckniveau angefahren und zugehörige Betriebsparameterwerte der Pumpe erfasst werden, wobei der Systemkompressibilitätswert auf Grundlage der Betriebsparameterwerte bestimmt wird.

13. Verfahren nach Anspruch 12, wobei das dritte und das vierte Druckniveau bevorzugt gleich dem ersten und zweiten Druckniveau sind.

14. Membranpumpenantrieb mit einem Drucksensor und mit einer Steuerung, wobei an eine Ankoppelfläche des Membranpumpenantriebs bevorzugt eine Pumpkassette ankoppelbar ist,
**dadurch gekennzeichnet,**
**dass** die Steuerung eine Funktion zur Durchführung eines Verfahrens zur Bestimmung eines Systemkompressibilitätswertes nach einem der Ansprüche 1 bis 10 und/oder zur Durchführung eines Verfahrens zur Bestimmung eines Luftanteils und/oder einer Luftmenge in der von dem Membranpumpenantrieb geförderten medizinischen Flüssigkeit nach einem der Ansprüche 11 bis 13 aufweist.

15. Blutbehandlungsmaschine, insbesondere Dialysemaschine, insbesondere Peritonealdialysemaschine, mit einem Membranpumpenantrieb nach Anspruch 14, wobei die Blutbehandlungsmaschine bevorzugt eine Pumpkassettenaufnahme und/oder ein Luftkissen zum Anpressen der Pumpkassette an eine Ankoppelfläche des Membranpumpenantriebs aufweist.

**Claims**

1. A method of determining a system compressibility value of a medical membrane pump drive, wherein a first and a second pressure level are moved to and a first and second operating parameter value of the membrane pump drive is detected, with the system compressibility value being determined on the basis of the detected operating parameter values,
**characterized in that**
the membrane of the membrane pump drive is supported at a rigid surface during the determination of the system compressibility value.

2. A method in accordance with claim 1, wherein the first and second pressure levels exceed the maximum counter-pressure of the membrane.

3. A method in accordance with claim 1 or claim 2, wherein a pump cassette can be coupled to the membrane pump drive; and wherein the determination of the system compressibility value takes place before the pump cassette has been coupled, with the membrane preferably contacting a receiving surface of a pump cassette receiver.

4. A method in accordance with claim 1 or claim 2, wherein a pump cassette can be coupled to the membrane pump drive; and wherein the determination of the system compressibility value takes place with a coupled pump cassette, with the membrane preferably being completely pressed into a pump chamber of the pump cassette and is supported at the rear wall of the pump chamber.

5. A method in accordance with claim 1 or claim 2, wherein the determination of the system compressibility value takes place at a vacuum, with the membrane preferably being completely drawn into a drive chamber of the membrane pump drive and contacting a rear wall of the drive chamber.

6. A method in accordance with one of the preceding claims, wherein an air cushion via which, in normal operation, a pump cassette can be pressed toward a coupling surface of the membrane pump drive is filled to operating pressure before determining the system compressibility value.

7. A method in accordance with one of the preceding claims, wherein the first pressure level is greater than 50 mbar (50 hPa), preferably greater than 75 mbar, further preferably greater than 100 mbar, further preferably greater than 150 mbar, and/or wherein the first pressure level is smaller than 600 mbar (600 hPa), preferably smaller than 400 mbar, further preferably smaller than 300 mbar, further preferably smaller than 250 mbar; and/or wherein the second pressure level is greater than 50 mbar (50 hPa), preferably greater than 100 mbar, further preferably greater than 200 mbar, further preferably greater than 250 mbar; and/or wherein the second pressure level is smaller than 600 mbar, preferably smaller than 500 mbar, further preferably smaller than 450 mbar, further preferably smaller than 400 mbar; and/or wherein the difference between the first and second pressure levels is greater than 5 mbar (5 hPa), preferably greater than 10 mbar, further preferably greater than 40 mbar, further preferably greater than 80 mbar and/or wherein the difference between the first and second pressure levels is smaller than 500 mbar (500 hPa), preferably smaller than 400 mbar, further preferably smaller than 300 mbar, further preferably smaller than 200 mbar.

8. A method in accordance with one of the preceding claims, wherein a system compressibility value is determined both at vacuum and at excess pressure.

9. A method in accordance with one of the preceding claims, wherein the system compressibility value depends on the difference of the operating parameter values that are determined at the first and second pressure levels, with the system compressibility value preferably depending on the difference between the pump positions that the membrane pump drive adopts at the first and second pressure levels and further preferably corresponding to this difference.

10. A method in accordance with one of the preceding claims, wherein the membrane pump drive has a drive chamber that is closed by the membrane, with the membrane being deflected outwardly out of the drive chamber by excess pressure in the drive chamber and being deflected inwardly into the drive chamber by vacuum in the drive chamber; and/or wherein a pressure sensor is provided that determines the pressure in the drive chamber to move to the first and second pressure levels; and/or wherein the pressure in the drive chamber is preferably produced via a piston-in-cylinder unit connected to the drive chamber; wherein a length sensor is preferably provided that detects the position of the piston as an operative parameter value; and/or wherein the transmission of the pressure onto the membrane preferably takes place hydraulically, with the piston-in-cylinder unit and the drive chamber preferably being filled with hydraulic fluid.

11. A method of determining an air proportion and/or an air quantity in a medical fluid conveyed by a membrane pump, for which purpose a first and a second pressure level are moved to by a corresponding control of a membrane pump drive of the membrane pump and associated operating parameter values of the membrane pump are detected, with the air proportion and/or the air quantity being determined on the basis of the operating parameter values, wherein a system compressibility value of the membrane pump drive is taken into account in the determination of the air proportion and/or of the air quantity, with the determination of the system compressibility value taking place in accordance with a method in accordance with one of the claims 1 to 10.

12. A method in accordance with claim 11, wherein a system compressibility value of the membrane pump drive is taken into account in the determination of the air proportion and/or of the air quantity, wherein a third and fourth pressure level are moved to for determining the system compressibility value and associated operating parameter values of the pump are detected, with the system compressibility value being determined on the basis of the operating parameter values.

13. A method in accordance with claim 12, wherein the third and fourth pressure levels are preferably equal to the first and second pressure levels.

14. A membrane pump drive having a pressure sensor and having a control, wherein a pump cassette can preferably be coupled to a coupling surface of the membrane pump drive,
**characterized in that**
the control has a function for carrying out a method of determining a system compressibility value in accordance with one of the claims 1 to 10 and/or for carrying out a method of determining an air proportion and/or an air quantity in the medical fluid conveyed by the membrane pump drive in accordance with one of the claims 11 to 13.

15. A blood treatment machine, in particular a dialysis machine, in particular a peritoneal dialysis machine, having a membrane pump drive in accordance with claim 14, wherein the blood treatment machine preferably has a pump cassette receiver and/or an air cushion for pressing the pump cassette to a coupling surface of the membrane pump drive.

**Revendications**

1. Procédé pour déterminer une valeur de compressibilité du système d'un entraînement de pompe médicale à membrane, un premier et un deuxième niveau de pression étant atteints et une première et une deuxième valeur de paramètre de fonctionnement de l'entraînement de pompe à membrane étant détectées, la valeur de compressibilité du système étant déterminée sur la base des valeurs de paramètre de fonctionnement détectées, **caractérisé en ce que**

la membrane de l'entraînement de pompe à membrane s'appuie sur une surface rigide pendant la détermination de la valeur de compressibilité du système.

2. Procédé selon la revendication 1, dans lequel le premier et le deuxième niveau de pression dépassent la contre-pression maximale de la membrane.

3. Procédé selon la revendication 1 ou 2, dans lequel une cassette de pompe peut être couplée à l'entraînement de pompe à membrane, et dans lequel la détermination de la valeur de compressibilité du système est effectuée avant que la cassette de pompe n'ait été couplée, la membrane s'appliquant de préférence sur une surface de réception d'un logement de cassette de pompe.

4. Procédé selon la revendication 1 ou 2, dans lequel une cassette de pompe peut être couplée à l'entraînement de pompe à membrane, et dans lequel la détermination de la valeur de compressibilité du système est effectuée avec la cassette de pompe couplée, la membrane étant de préférence entièrement poussée à l'intérieur d'une chambre de pompe de la cassette de pompe et s'appuyant sur la paroi arrière de la chambre de pompe.

5. Procédé selon la revendication 1 ou 2, dans lequel la détermination de la valeur de compressibilité du système est effectuée sous dépression, la membrane étant de préférence entièrement tirée à l'intérieur d'une chambre d'entraînement de l'entraînement de pompe à membrane et s'appliquant sur une paroi arrière de la chambre d'entraînement.

6. Procédé selon l'une des revendications précédentes, dans lequel, avant la détermination de la valeur de compressibilité du système, un coussin d'air, par le biais duquel une cassette de pompe est, en fonctionnement normal, pressée contre une surface de couplage de l'entraînement de pompe à membrane, est rempli à la pression de fonctionnement.

7. Procédé selon l'une des revendications précédentes, dans lequel le premier niveau de pression est supérieur à 50 mbar (50 hPa), de préférence supérieur à 75 mbar, de préférence encore supérieur à 100 mbar, de préférence encore supérieur à 150 mbar, et/ou dans lequel le premier niveau de pression est inférieur à 600 mbar (600 hPa), de préférence inférieur à 400 mbar, de préférence encore inférieur à 300 mbar, de préférence encore inférieur à 250 mbar, et/ou dans lequel le deuxième niveau de pression est supérieur à 50 mbar (50 hPa), de préférence supérieur à 100 mbar, de préférence encore supérieur à 200 mbar, de préférence encore supérieur à 250 mbar, et/ou dans lequel le deuxième niveau de pression est inférieur à 600 mbar, de préférence inférieur à 500 mbar, de préférence encore inférieur à 450 mbar, de préférence encore inférieur à 400 mbar, et/ou dans lequel la différence entre le premier et le deuxième niveau de pression est supérieure à 5 mbar (5 hPa), de préférence supérieure à 10 mbar, de préférence encore supérieure à 40 mbar, de préférence encore supérieure à 80 mbar, et/ou dans lequel la différence entre le premier et le deuxième niveau de pression est inférieure à 500 mbar (500 hPa), de préférence inférieure à 400 mbar, de préférence encore inférieure à 300 mbar, de préférence encore inférieure à 200 mbar.

8. Procédé selon l'une des revendications précédentes, dans lequel une valeur de compressibilité du système est déterminée aussi bien sous dépression que sous surpression.

9. Procédé selon l'une des revendications précédentes, dans lequel la valeur de compressibilité du système dépend de la différence entre les valeurs de paramètre de fonctionnement, qui sont déterminées au premier et au deuxième niveau de pression, la valeur de compressibilité du système dépendant de préférence de la différence entre les positions de pompe, que l'entraînement de pompe à membrane adopte au premier et au deuxième niveau de pression, et correspondant de préférence encore à cette différence.

10. Procédé selon l'une des revendications précédentes, dans lequel l'entraînement de pompe à membrane comporte une chambre d'entraînement, qui est fermée par la membrane, la membrane étant déviée vers l'extérieur depuis la chambre d'entraînement par une surpression dans la chambre d'entraînement et vers l'intérieur dans la chambre d'entraînement par une dépression dans la chambre d'entraînement, et/ou dans lequel un capteur de pression est prévu, qui détermine la pression dans la chambre d'entraînement, pour atteindre le premier et le deuxième niveau de pression, et/ou dans lequel la pression dans la chambre d'entraînement est de préférence générée par le biais d'une unité à piston et cylindre se trouvant en liaison avec la chambre d'entraînement, un capteur de distance étant de préférence prévu, qui détecte la position du piston en tant que valeur de paramètre de fonctionnement, et/ou dans lequel la transmission de la pression sur la membrane est effectuée de préférence de manière hydraulique, l'unité à piston et cylindre et la chambre d'entraînement étant de préférence remplies de fluide hydraulique.

11. Procédé de détermination d'une part d'air et/ou d'une quantité d'air dans un liquide médical transporté par une pompe à membrane, pour lequel un premier et un deuxième niveau de pression sont atteints par une commande correspondante d'un entraînement de pompe à membrane de la pompe à membrane et des valeurs de paramètre de fonctionnement de la pompe à membrane sont détectées, la part d'air et/ou la quantité d'air étant déterminée sur la base des valeurs de paramètre de fonctionnement, une valeur de compressibilité du système de l'entraînement de pompe à membrane étant prise en compte lors de la détermination de la part d'air et/ou de la quantité d'air, la détermination de la valeur de compressibilité du système étant effectuée d'après un procédé selon l'une des revendications 1 à 10.

12. Procédé selon la revendication 11, dans lequel une valeur de compressibilité du système de l'entraînement de la pompe à membrane est prise en compte lors de la détermination de la part d'air et/ou de la quantité d'air, un troisième et un quatrième niveau de pression étant atteints pour la détermination de la valeur de compressibilité du système et des valeurs de paramètre de fonctionnement associées de la pompe étant détectées, la valeur de compressibilité du système étant déterminée sur la base des valeurs de paramètre de fonctionnement.

13. Procédé selon la revendication 12, dans lequel le troisième et le quatrième niveau de pression sont de préférence égaux au premier et au deuxième niveau de pression.

14. Entraînement de pompe à membrane comprenant un capteur de pression et un dispositif de commande, une cassette de pompe pouvant de préférence être couplée à une surface de couplage de l'entraînement de pompe à membrane,
**caractérisé en ce que**
le dispositif de commande comporte une fonction pour l'exécution d'un procédé de détermination d'une valeur de compressibilité du système selon l'une des revendications 1 à 10 et/ou pour l'exécution d'un procédé de détermination d'une part d'air et/ou d'une quantité d'air dans le liquide médical transporté par l'entraînement de pompe à membrane selon l'une des revendications 11 à 13.

15. Machine de traitement du sang, en particulier machine de dialyse, en particulier machine de dialyse péritonéale, comprenant un entraînement de pompe à membrane selon la revendication 14, la machine de traitement du sang comportant de préférence un logement de cassette de pompe et/ou un coussin d'air pour presser la cassette de pompe contre une surface de couplage de l'entraînement de pompe à membrane.

Fig. 1

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2013176770 A2
- DE 19919572 A1
- DE 102011105824 B3